# EUROPÄISCHE PATENTSCHRIFT

(11) **EP 0 787 466 B1**
(45) Veröffentlichungstag und Bekanntmachung des Hinweises auf die Patenterteilung: **03.11.1999**
(21) Anmeldenummer: 97100715.8
(22) Anmeldetag: 17.01.1997
(51) Int. Cl.: A61B 17/68

(54) **Mittel zur Fixierung eines kalottenförmigen Schädelkapselsegments**
Device for securing a cranial piece
Elément de fixation d'un fragment de la calotte cranienne

(30) Priorität: 03.02.1996 DE 19603887
(43) Veröffentlichungstag der Anmeldung: 06.08.1997
(60) Teilanmeldung: 99103138.6
(73) Patentinhaber: Lerch, Karl-Dieter, Dr. med., D-58452 Witten (DE)
(72) Erfinder: Lerch, Karl-Dieter, Dr. med., D-58452 Witten (DE)
(74) Vertreter: Henfling, Fritz, Dipl.-Ing.

(56) Entgegenhaltungen:
- DE-B- 2 125 556
- DE-U- 29 614 921
- US-A- 2 576 649

## Beschreibung

Die Erfindung betrifft eine Anordnung zum Fixieren eines aus der Schädelkapsel zum Zwecke des operativen Eingriffs herausgetrennten Knochenstücks nach der Operation am verbliebenen Schädelbein.

Bei Hirnoperationen bedarf es vielfach der Entfernung eines Knochenstücks aus der Schädelkapsel, um dem Operateur den Zugang zum Operationsbereich zu eröffnen. Das vorweg aus der Schädelkapsel herausgesägte Knochenstück muß im Anschluß an die Operation wieder in die Schädelkapsel eingefügt und der Kapsel gegenüber fixiert werden. Die Fixierung des Knochenstücks gegenüber der Schädelkapsel erfolgt bislang durch eine Zusammenfassung des Knochenstücks mit dem verbliebenen Schädelbein durch das Knochenstück und das Schädelbein durchsetzende Stahldrahtschlingen, deren über die Schädelkapsel nach dem Setzen der Drähte vorspringende Enden miteinander verdrillt werden. Hierbei handelt es sich jedoch um eine relativ instabile Zusammenfassung des Knochenstücks mit dem verbliebenen Schädelbein, die das Verwachsen des Knochenstücks mit dem verbliebenen Schädelbein beeinträchtigt und dann auch zu Entzündungen der Kopfhaut führen kann. Darüber hinaus erweist sich diese Art und Weise der Zusammenfassung des Knochenstücks mit dem verbliebenen Schädelbein insofern als nachteilig, als die Stahldrähte beispielsweise bei der postoperativen computertomographischen Kontrolle erhebliche Bildstörungen hervorrufen, die eine sichere Beurteilung der Weichteilstrukturen des Hirns beeinträchtigen. Der Ersatz der Stahldrähte durch nichtresorbierbare körperverträgliche Fäden behebt den zuletztgenannten Nachteil zwar, in Kauf genommen werden muß dabei allerdings eine noch instabilere Fixierung des Knochenstücks gegenüber dem verbliebenen Schädelbein. Die Zusammenfassung des Knochenstücks mit dem verbliebenen Schädelbein mit Hilfe von den Stoß zwischen Knochenstück und Schädelbein überdeckenden, einerseits mit dem Knochenstück und andererseits mit dem verbliebenen Schädelbein verschraubbaren Plättchen aus einem körperverträglichen Metall, wie Titan, die dann auch vor dem Trennschnitt in der Schädelkapsel gesetzte Bohrungen verschließen (EP-A-0 510 390), stellt auch noch keine befriedigende Lösung dar, zumal es sich hierbei um eine umständliche sowie zeitaufwendige und damit kostenintensive Fixierung handelt.

Der nächstliegende Stand der Technik ist aus der US-A-2 576 649 bekannt. Diese offenbart eine einstückige Schädelklammer mit zwei gegenüberliegenden die Schädeldecke aufnehmenden Klauen.

Ausgehend vom Stand der Technik lag der Erfindung die Aufgabe zugrunde, die Möglichkeit einer einfacheren, mit möglichst geringem Zeitaufwand zu realisierenden Zusammenfassung eines für einen operativen Eingriff aus der Schädelkapsel herausgetrennten Knochenstücks nach dem operativen Eingriff gegenüber dem verbliebenen Schädelbein zu schaffen, die dann auch die gewünschte eindeutige und anhaltende Lagefixierung erbringt.

Die Aufgabe wird mit einer Anordnung zum Fixieren eines aus der Schädelkapsel zum Zwecke eines operativen Eingriffs herausgetrennten Knochenstückes am verbliebenen Schädelbein, bestehend aus einem an einem Ende mit einem Flachkopf versehenen Stift, einer am Flachkopf zur Anlage kommenden gewölbten ersten Scheibe und einer gewölbten zweiten Scheibe, die aus einem körperverträglichen Metall oder einer körperverträglichen metallischen Verbindung hergestellt sind, gelöst, wobei die Scheiben im Randbereich der konkaven Seite mit Zacken sowie in der Mitte mit einer Bohrung versehen sind, die an den Schaft des Stiftes angepaßt ist, auf den die Scheiben die Zacken einander zugewandt, aufsteckbar sind und an dem die zweite Scheibe festlegbar ist.

Die im vorausgehenden definierte erfindungsgemäße Fixierungsanordnung wird in der Weise angesetzt, daß die Stifte mit ihnen bereits zugeordneter stiftkopfseitiger Scheibe durch eine auf den Durchmesser der Scheibe ausgelegte Aussparung, in der Regel im herausgetrennten Knochenstück, unter die Knochenpartien, der Schaft des Stiftes sich in der Stoßfuge zwischen den Knochenpartien führend, beabstandet über den Umfang des Knochenstücks verteilt werden, auf die aus der Schädelkapsel vorspringenden Stifte sodann die zweite Scheibe aufgesteckt und die zweite Scheibe eine Verkrallung sowohl der ersten als auch der zweiten Scheibe mit den Randbereichen einerseits des verbliebenen Schädelbeins und andererseits des wieder zu integrierenden Knochenstücks angestellt sowie gegenüber dem Stift festgelegt wird.

Ausgestaltungen des erfindungsgemäßen Fixierungsmittel ergeben sich aus den Unteransprüchen 2 bis 7. Als körperverträgliches Metall bietet sich in erster Linie Titan an oder aber eine geeignete Titanverbindung, etwa Ti₆ A₆ Va. Ein Fixierungsmittel auf Titanbasis erweist sich deshalb als vorteilhaft, weil es die postoperative computermonografische Kontrolle nicht beeinträchtigt. Zur Lagestabilisierung der voreilend auf den Stift aufzusteckenden Scheibe trägt bei, wenn der Übergang vom Kopf des Stiftes in den Schaft konisch mit der Maßgabe ausgebildet ist, das daraus Preßsitz der Scheibe auf dem Stift bei gegen den Kopf des Stiftes anliegender Scheibe resultiert. In Zusammenhang damit kann vorgesehen sein, daß vom Durchgang der voreilend auf den Stift aufsteckbaren Scheibe radial verlaufende Anschnitte ausgehen, weitergehend kann vorgesehen sein daß die Scheibe mittig begrenzt eingesenkt ist. Dann auch aus Gründen der Materialersparnis können in den Scheiben zwischen Durchgang und Rand über den Umfang verteilt in regelmäßigen Abständen Aussparungen vorgesehen sein. Die Festlegung des Fixierungsmittels gegenüber den zusammenzufassenden Knochenpartien kann in einer Weise geschehen, die im Prinzip dem Blindnietverfahren entspricht. Hierfür können am Schaft des Stiftes Rasten ausgebildet sein, mit denen die nacheilend auf den Schaft aufsteckbare Scheibe in Richtung auf den Kopf des Stiftes angezogenen Formschluß einzugehen vermag. Erfährt die nacheilend auf den Schaft des Stiftes aufgesteckte Scheibe eine zu ihrer Wölbung gegenläufige Verformung, kommt es allein schon dadurch zu einer Festlegung der Scheibe auf dem Stift, durch Klemmung. Möglich ist auch die Ausführung des Schaftes als Gewindestift, an dem eine Mutter ansetzbar ist, die gegen die nacheilend auf den Stift aufsteckbare Scheibe zur Anlage kommend angezogen wird. In allen Fällen wird die nacheilend auf den Schaft gesteckte Scheibe bis zum Einsenken der an den Scheiben ausgebildeten Zacken in die zusammenzufassenden Knochenpartien verlagert, woraus sich die gewünschte Fixierung des wieder in die Schädelkapsel zu integrierenden Knochenstücks gegenüber den angrenzenden Partien der Schädelkapsel ergibt.

Das neue Fixierungsmittel läßt sich leicht und ohne größeren Zeitaufwand handhaben und erbringt die gewünschte, eindeutige und anhaltende Fixierung des wieder in die Schädelkapsel zu integrierenden, für einen operativen Eingriff aus der Schädelkapsel herausgetrennten Knochenstücks.

In der Zeichnung ist die Erfindung weitergehend erläutert. Es zeigen:
- Figur 1: einen Längsschnitt durch das in Sprengarstellung dargestellt Fixierungsmittel,
- Figur 2: eine Ausführungsform der voreilend auf den Stift aufzubringenden Scheibe in Richtung des Pfeiles II in Figur 1,
- Figur 3: eine Ausführungsform der nacheilend auf den Stift aufzubringenden Scheibe in Richtung des Pfeiles III in Figur 1,
- Figur 4: die zusammengesetzten Elemente des Fixierungsmittels im Längsschnitt,
- Figur 5: eine den Einsatz des neuen Fixierungsmittels demonstrierende Darstellung,
- Figur 6: einen Schnitt nach Linie VI - VI in Figur 5.

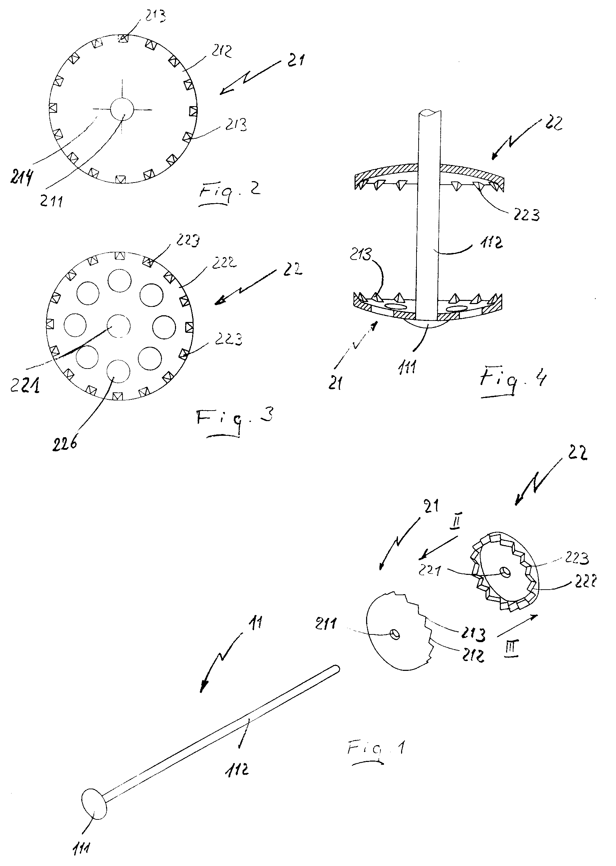

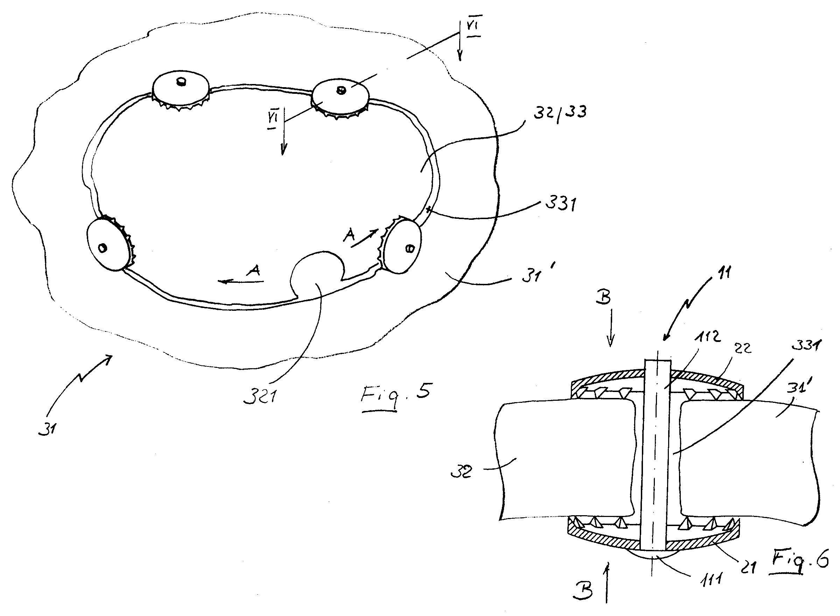

Das Mittel zur Fixierung eines aus der Schädelkapsel herausgetrennten Knochenstücks gegenüber dem verbliebenen Schädelbein nach operativem Eingriff besteht aus dem Stift 11 mit dem Kopf 111 und dem Schaft 112, der vorweg auf den Schaft 112 des Stiftes 11 aufzubringenden, von innen gegen das verbliebene Schädelbein und das damit wieder zusammenzufassende Knochenstück zur Anlage kommenden Scheibe 21 und der dann auch noch auf den Schaft 112 des Stiftes 11 aufzubringenden, von außen gegen das Schädelbein und das damit wieder zusammenzufassende Knochenstück zur Anlage kommenden Scheibe 22. Beide Scheiben sind mittig gelocht, durch die Löcher 221 und 222 erstreckt sich im zusammengesetzten Zustand der Elemente des Fixierungsmittels der Schaft 112 des Stiftes 11. Die Ränder (212, 222) der gewölbten Scheiben 21, 22 sind des weiteren unter Ausbildung von Zacken 213, 223 gezackt. Wie das in Figur 1 angedeutet und aus Figur 4 entnehmbar ist, werden die Scheiben 21 und 22 die gezackten Ränder 212, 222 einander zugewandt auf den Schaft 112 des Stiftes 11 aufgebracht.

Von dem mittigen bezüglich des Querschnitts des Stiftschaftes 112 paßgerechten Durchgangsloch 211, in der Scheibe 21 ausgehende Radialschnitte, angedeutet in Figur 2, dort die Bezugsziffer 214, erbringen bei konischem Übergang vom Kopf 111 des Stiftes 11 in dessen Schaft 112 in wünschenswerter Weise eine eindeutige Zuordnung der Scheibe 21 zum Stift 11, mit der beim Setzen des Fixierungselements das verbliebene Schädelbein und das damit wieder zusammenzufassende Knochenstück unterfahren wird. Die Scheiben 21, 22 können dann auch material- und gewichtssparend im Bereich zwischen Durchgang und Rand über den Umfang verteilt mit Lochungen versehen sein, wie das in Figur 3 angedeutet ist (dort die Bezugsziffer 226). Beide Ausgestaltungen, also die von den Durchgangslöchern ausgehende Anschnitte und die materialsparenden Lochungen, können dann auch gemeinsam vorgesehen sein.

Aus den Figuren 5 und 6 ist die Einsatzweise des neuen Fixierungsmittels zu entnehmen. In Figur 5 ist mit 31 die gewachsene Schädelkapsel bezeichnet, aus der zwecks Ausbildung eines Zugangs zum Gehirn das Knochenstück 32 herausgefräst wurde. Nach Entfernen des Knochenstücks 32 ist das unter der Schädelkapsel liegende Gehirn in diesem Bereich 33 für einen operativen Eingriff zugänglich. Im Anschluß an den vollzogenen Eingriff wird das an seinem Umfang mit einem der Größe der Bestandteil des Fixierungsmittels bildenden, von innen gegen das verbliebene Schädelbein 31' und das damit wieder zusammenzufassende Knochenstück 32 zur Anlage kommenden Scheibe 22 entsprechenden Rücksprung 321 zuzüglich geringfügigen Übermaßes versehene Knochenstück 32 lageorientiert wieder in den freigelegten Bereich 33 der Schädelkapsel eingefügt und aufeinander folgend die Bestandteil des Fixierungsmittels bildenden Stifte 11 mit bereits auf deren Schaft 112 aufgebrachter, von innen gegen das verbliebene Schädelbein 31' und das wieder eingesetzte Knochenstück 32 zur Anlage kommender Scheibe 22 durch den in dem Knochenstück 32 eingearbeiteten Rücksprung 321 in die aus der Fräsung hervorgegangene Stoßfuge 331 überführt und in der Stoßfuge 331 an die Stelle überführt an der jeweils die Fixierung des Knochenstücks 32 gegenüber dem verbliebenen Schädelbein 31' erfolgen soll (Pfeile A in Fig. 5). An Ort und Stelle wird sodann die zweite, von außen gegen das verbliebene Schädelbein 31' und das damit zusammenzufassende Knochenstück 32 zur Anlage kommende Scheibe 22 auf den Schaft 112 des Stiftes 11 aufgeschoben und zur Anlage gegen das verbliebene Schädelbein 31' und das damit zusammenzufassende Knochenstück 32 gebracht. Am Überstand des Stiftschaftes 112 über die äußere Scheibe 22 wird sodann ein Werkzeug angesetzt, etwa ein Werkzeug nach Art eines Werkzeuges zum Setzen von Blindnieten, mit dem das Fixierungsmittel unter Einsenken der an den Rändern der Scheiben 21, 22 ausgebildeten Zacken 213, 223 in das verbliebene Schädelbein 31' und das Knochenstück 32 gesetzt wird (Pfeile B in Fig. 6). Anschließend wird der danach noch verbleibende Überstand des Stiftschaftes 112 über die äußere Scheibe 22 abgetrennt. Das im vorausgehenden detailliert beschriebene Setzen mit Hilfe eines Werkzeuges nach Art eines Werkzeuges zum Setzen von Blindnieten schließen eine Verklammerung der das verbliebene Schädelbein und das wieder eingesetzte Knochenstück zusammenfassenden Scheiben des Fixierungselementes mit dem Schädelbein und dem Knochenstück über eine Schraubverbindung nicht aus. Dafür ist der Schaft des Bestandteil des Fixierungsmittels bildenden Stiftes dann als Gewindestift ausgebildet, auf dessen Gewinde eine gegen die von außen gegen das verbliebene Schädelbein und das damit wiederum zusammenzufassende Knochenstück zur Anlage kommende, Bestandteil des Fixierungsmittels bildende Scheibe gegen Schädelbein und Knochenstück bis zur Verkrallung beider Scheiben im Schädelbein und am Knochenstück angezogen wird.

Mit solchermaßen über den Umfang des mit dem verbliebenen Schädelbein 31' wieder zusammenzufassenden Knochenstücks 32 verteilt angesetzten erfindungsgemäßen Fixierungsmitteln, wird das Knochenstück 32 gegenüber dem verbliebenen Schädelbein 31' - wie gewünscht - eindeutig fixiert.

## Patentansprüche

1. Anordnung zum Fixieren eines aus der Schädelkapsel zum Zwecke eines operativen Eingriffs herausgetrennten Knochenstückes am verbliebenen Schädelbein, bestehend aus einem an einem Ende mit einem Flachkopf (111) versehenen Stift (11), einer am Flachkopf (111) zur Anlage kommenden gewölbten ersten Scheibe (21) und einer gewölbten zweiten Scheibe (22), die aus einem körperverträglichen Metall oder einer körperverträglichen metallischen Verbindung hergestellt sind, wobei die Scheiben (21, 22) im Randbereich der konkaven Seite mit Zacken (213, 223) sowie in der Mitte mit einer Bohrung (211, 221) versehen sind, die an den Schaft (112) des Stiftes (11) angepaßt ist, auf den die Scheiben (21, 22) die Zacken (213, 223) einander zugewandt, aufsteckbar sind und an dem die zweite Scheibe (22) festlegbar ist.

2. Anordnung nach Anspruch 1, dadurch gekennzeichnet, daß der Stift (11) und die Scheiben (21, 22) aus Titan oder einer körperverträglichen Titanverbindung bestehen.

3. Anordnung nach Anspruch 1 oder Anspruch 2, gekennzeichnet durch von der Bohrung (211) der ersten Scheibe (21) ausgehende Radialschnitte (214).

4. Anordnung nach einem der Ansprüche 1 bis 3, dadurch gekennzeichnet, daß die erste Scheibe (21) mittig gegenläufig zu ihrer Wölbung eingesenkt ist.

5. Anordnung nach einem der Ansprüche 1 bis 4, dadurch gekennzeichnet, daß die Scheiben (21, 22) im Bereich zwischen Bohrung (211, 221) und Rand über den Umfang verteilt in regelmäßigen Abständen mit weiteren Bohrungen (226) versehen sind.

6. Anordnung nach einem der Ansprüche 1 bis 5, gekennzeichnet durch am Schaft (112) des Stiftes (11) ausgebildete, die auf den Schaft des Stiftes aufgesteckte, in Richtung auf den Kopf (111) des Stiftes (11) verlagerte zweite Scheibe (22) hinterfangende Rasten.

7. Anordnung nach einem der Ansprüche 1 bis 5, dadurch gekennzeichnet, daß der Schaft (112) des Stiftes (11) als Gewindestange ausgebildet ist, auf die eine gegen die zweite Scheibe (22) zur Anlage kommende Mutter aufschraubbar ist.

## Claims

1. Arrangement for securing a piece of bone, which has been removed from the skull cap for the purpose of an operation, on the remaining skull, comprising a pin (11), which is provided with a flat head (111) on one end, a curved first disc (21), which abuts against the flat head (111), and a curved second disc (22), which discs are produced from a metal which is compatible with the body or from a metallic compound which is compatible with the body, wherein the discs (21, 22) are provided with teeth (213, 223) in the edge region of the concave side and with a bore (211, 221) in the centre, said bore being adapted to the shaft (112) of the pin (11), on which pin the discs (21, 22) are mountable, the teeth (213, 223) facing one another, and on which pin the second disc (22) is securable.

2. Arrangement according to claim 1, characterised in that the pin (11) and the discs (21, 22) are formed from titanium or a titanium compound which is compatible with the body.

3. Arrangement according to claim 1 or claim 2, characterised by radial cuts (214), which extend from the bore (211) of the first disc (21).

4. Arrangement according to one of claims 1 to 3, characterised in that the first disc (21) is inserted centrally in the opposite direction to its curvature.

5. Arrangement according to one of claims 1 to 4, characterised in that the discs (21, 22) are provided with additional bores (226) in the region between bore (211, 221) and edge at regular spacings distributed over the circumference.

6. Arrangement according to one of claims 1 to 5, characterised by locking means, which are provided on the shaft (112) of the pin (11) and engage behind the second disc (22), which is mounted on the shaft of the pin and is displaced in the direction of the head (111) of the pin (11).

7. Arrangement according to one of claims 1 to 5, characterised in that the shaft (112) of the pin (11) is configured as a threaded rod, on which there can be screwed one nut, which abuts against the second disc (22).

## Revendications

1. Dispositif de fixation d'une partie d'un os, qui a été sectionnée de la boîte crânienne en vue d'une intervention chirurgicale, sur l'os crânien restant, constitué d'une broche (11) pourvue à une extrémité d'une tête plate (111), d'un premier disque (21) bombé venant s'appliquer contre la tête plate (111) ainsi qu'un deuxième disque (22) bombé, lesquels disques sont fabriqués dans un métal toléré par le corps ou en un alliage métallique toléré par le corps, les disques (21, 22) étant pourvus de dents (213, 223), dans la zone de bordure de la face concave, ainsi qu'au milieu, d'un perçage (211, 221) qui est adapté à la tige (112) de la broche (11), sur laquelle les disques (21, 22), les dents (213, 223) tournées l'une vers l'autre peuvent être enfichés et à laquelle le deuxième disque (22) peut être fixé.

2. Dispositif selon la revendication 1, caractérisé en ce que la broche (11) et les disques (21, 22) sont en titane ou en alliage de titane toléré par le corps.

3. Dispositif selon la revendication 1 ou la revendication 2, caractérisé par des entailles radiales (214) partant du perçage (211) du premier disque (21).

4. Dispositif selon l'une des revendications 1 à 3, caractérisé en ce que le premier disque (31) est encastré au milieu dans le sens contraire à son bombement.

5. Dispositif selon l'une des revendications 1 à 4, caractérisé en ce que les disques (21, 22) sont pourvus, dans la zone comprise entre le perçage (211, 221) et le bord, de perçages (226) complémentaires répartis à distances régulières sur le pourtour.

6. Dispositif selon l'une des revendications 1 à 5, caractérisé par des encoches formées sur la tige (112) de la broche (11), qui saisissent de l'arrière le deuxième disque (22) enfiché sur la tige de la broche et déplacé dans la direction de la tête (111) de la broche (11).

7. Dispositif selon l'une des revendications 1 à 5, caractérisé en ce que la tige (112) de la broche (11) est réalisée sous la forme d'une tige filetée sur laquelle peut être vissé un écrou venant s'appliquer contre le deuxième disque (22).
